# EUROPEAN PATENT APPLICATION

(11) **EP 4 606 331 A1**
(43) Date of publication of application: **27.08.2025**
(21) Application number: 25156282.3
(22) Date of filing: 06.02.2025
(51) Int. Cl.: A61B 18/02, A61B 34/10

(54) **TEMPERATURE VOLUME HISTOGRAM GENERATION APPARATUS AND METHOD**

(30) Priority: 21.02.2024 US 202418583485
(71) Applicant: Varian Medical Systems, Inc., Palo Alto, CA 94304 (US)
(72) Inventor: BOIVIN, Gael, 8032 Zurich (CH); MEDEMA, Ryan, Georgetown, 78633 (US); VALLAPUREDDY, Vineel, Plymouth, 55446 (US); KRATZKE, Lisa, 91054 Erlangen (DE); JOY, Vaisakh, 91052 Erlangen (DE); WILKINSON, Anthony, Mid-Levels (HK); SHARMA, Ricky, Lightwater, GU18 5YQ (GB); HAHN, David, Chicago, 60642 (US)
(74) Representative: Mathisen & Macara LLP

(57) **Abstract**

To facilitate administering thermal ablation (such as, but not limited to, cryotherapy) to a patient's target volume, a control circuit can access characterizing information for a particular thermal ablation apparatus and then generate a temperature volume histogram as a function of the characterizing information for that particular thermal ablation apparatus.

## Description

### Technical Field

These teachings relate generally to generation of temperature volume histograms to improve treating a patient's planning target volume with thermal energy.

### Background

The use of thermal energy to treat medical conditions comprises a known area of prior art endeavor. For example, thermal ablation can comprise a component of a treatment plan for reducing or eliminating unwanted tumors. The latter can comprise, for example, exposing a targeted tissue to a lethal low temperature. So-called cryo-needles/probes can be employed to inject cold materials at targeted locations within a patient to achieve the desired results.

Thermal ablation can be performed by a surgeon and interventional radiologist. Preparation for the procedure is often informed by patient imaging that helps the practitioner estimate an entry route through which the cryo-needle/probe should be inserted as well as the selection of a particular needle/probe size and/or the number of needles/probes required to eradicate the target. These choices can be influenced by such things as the patient's overall anatomy, medical guidelines, the practitioner's experience, and by the volume and the shape of the target.

Many practitioners consider that a lethal isotherm for a non-cancerous tissue is - 20 degrees Celsius and for cancerous tissue is -40 degrees Celsius. Regardless, and unfortunately, the actual tissue temperature cannot be practically measured empirically during a clinical intervention, as doing so would require multiple thermal recorders to be inserted in and around the target volume during the procedure.

Instead, practitioners typically rely upon imaging during (and after) the procedure (using, for example, computed tomography imaging and/or ultra sound imaging) to evaluate such things as needle positioning, the size and/or extent of the ice ball(s) generated during the procedure or the expected heated volume in the context of heat-mediated thermal ablation, and eventually the resultant liquified volume after the intervention. While certainly useful, current approaches in these regards are not wholly satisfactory in any number of ways. For instance, the ice ball corresponds to the 0 degree Celsius and less isotherm, but is not informative of the -20 or -40 degree Celsius isotherm that corresponds to the "lethal ice" isotherms.

### Brief Description of the Drawings

The above needs are at least partially met through provision of the temperature volume histogram generation apparatus and method described in the following detailed description, particularly when studied in conjunction with the drawings, wherein:
FIG. 1 comprises a block diagram as configured in accordance with various embodiments of these teachings;
FIG. 2 comprises a flow diagram as configured in accordance with various embodiments of these teachings;
FIG. 3 comprises a schematic representation as configured in accordance with various embodiments of these teachings;
FIG. 4 comprises an isotherm map as configured in accordance with various embodiments of these teachings;
FIG. 5 comprises a selected portion of the isotherm map presented in FIG. 4;
FIG. 6 comprises a schematic view as configured in accordance with various embodiments of these teachings;
FIG. 7 comprises a schematic view as configured in accordance with various embodiments of the invention;
FIG. 8 comprises a temperature volume histogram as configured in accordance with various embodiments of these teachings; and
FIG. 9 comprises a flow diagram as configured in accordance with various embodiments of the invention.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

### Detailed Description

Generally speaking, these various embodiments can serve to facilitate improvements in administering thermal ablation (such as, but not limited to, cryotherapy) to a patient's target volume. By one approach, a control circuit accesses characterizing information for a particular thermal ablation apparatus and then generates a temperature volume histogram as a function of the characterizing information for the particular thermal ablation apparatus. In an embodiment, the control circuit may access characterizing information of a plurality of different thermal ablation apparatuses. By another approach there is provided an apparatus to improve administration of thermal ablation to a patient's target volume comprising a memory having stored therein characterizing information for a particular thermal ablation apparatus, a control circuit operably coupled to the memory and configured to: access the characterizing information for the particular thermal ablation apparatus, and generate a temperature volume histogram as a function of the characterizing information for the particular thermal ablation apparatus. The thermal ablation apparatus may be a thermal ablation needle. The apparatus may comprise a thermal ablation treatment platform, which may comprise a thermal ablation console. The thermal ablation console may be operably coupled to a remote user interface. The control circuit may be integral with the thermal ablation console. Alternatively, or in combination therewith, the thermal ablation console may be operably coupled to an external control circuit. The control circuit may be operably coupled to a memory, which memory may be integral to the control circuit or may be physically discrete from the control circuit. This memory can also be local with respect to the control circuit or can be partially or wholly remote with respect to the control circuit. The control circuit can also operably couple to a network interface. In some embodiments, the control circuit may be operably coupled to a thermocouple array and/or to a data acquisition circuit.

These teachings will accommodate a variety of different kinds of characterizing information. By way of example, by one approach the characterizing information for the particular thermal ablation apparatus can include at least one of a thermal fluid that effects a desired thermal ablation result, a flow rate of the thermal fluid, and/or at least one physical dimension of the particular thermal ablation apparatus. For example, the characterizing information can comprise information that characterizes a particular thermal ablation needle/probe (such as characterizing information for a particular thermal ablation needle/probe from amongst a plurality of different candidate thermal ablation needles/probes).

The temperature volume histogram can be presented in any suitable manner. In one approach, the temperature volume histogram can be presented as a differential temperature volume histogram. By another approach, the temperature volume histogram can be presented as a cumulative temperature volume histogram. By one approach, generating the aforementioned temperature volume histogram can further comprise generating the temperature volume histogram as a further function of stored empirically-sensed temperature information. As one illustrative approach in those regards, that stored empirically-sensed temperature information can correspond, at least in part, to thermal results of the particular thermal ablation apparatus in a proxy medium.

These teachings are highly flexible in practice and will accommodate various modifications and/or supplemental features. As one illustrative example in those regards, the control circuit can be further configured to present, via a user interface, a graphic depiction of the aforementioned temperature volume histogram. As another illustrative example, the control circuit can be further configured to determine a zone of lethality as a function of the temperature volume histogram. The control circuit may be further configured to generate the temperature volume histogram by generating the temperature volume histogram as a further function of modeling content. The modeling content may represent at least one of patient thermal states, patient blood flow, and patient anatomy. The control circuit may be further configured to generate the temperature volume histogram by generating the temperature volume histogram as a further function of information regarding a patient's geometry. The information regarding the patient's geometry may comprise at least one of a specified patient volume of interest, a particular thermal ablation needle orientation/location/depth with respect to patient geometry, and/or a particular plan or pattern of administering and using treatment accoutrements.

So configured, these teachings can serve to better predict and/or model isothermic distribution across a target volume, for a given cryo-needle/probe (or combination of cryo-needles/probes) selection, both before, during, and/or after a corresponding thermal ablation treatment. These teachings can also facilitate intra and/or inter-clinic practice comparisons, independent of the tumor shape or absolute volume. It will be further appreciated that these teachings can provide a useful basic metric for recording the isotherm actually delivered during a thermal ablation procedure and to also facilitate comparing that result to an initially prescribed isotherm(s) and thereby help to ensure the overall quality of the treatment procedure and the patient follow-up.

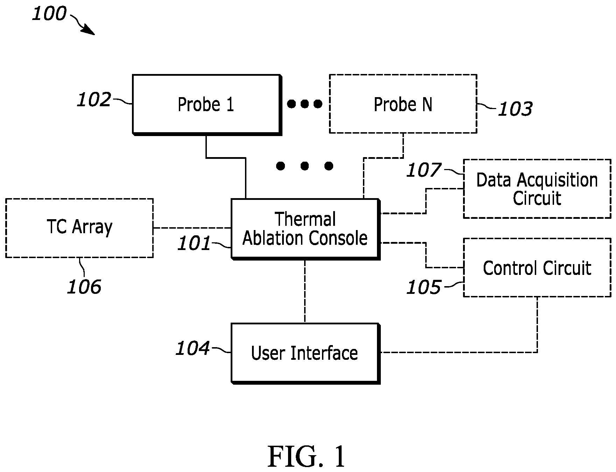

These and other benefits may become clearer upon making a thorough review and study of the following detailed description. Referring now to the drawings, and in particular to FIG. 1, an illustrative apparatus 100 that is compatible with many of these teachings will first be presented.

In this particular example, and for the sake of an illustrative non-limiting example, the enabling apparatus comprises a thermal ablation treatment platform 100. And again for the sake of an illustrative example, the following description will often presume this thermotherapy treatment platform 100 to comprise a cryotherapy treatment platform.

In this example, the cryotherapy treatment platform comprises a thermal ablation console 101 (such as, for example a CryoCare Touch 38T console as manufactured by Varian). Such a system is configured to freeze/ablate a selected patient's tissue by the application of extreme cold temperatures, typically via the administration of a cold substance via one or more corresponding needles/probes (shown as probe 1 through probe N (where N is an integer greater than "1"), the latter denoted by reference numerals 102 and 103, respectively).

The thermal ablation console 101 may have an integral user interface including, for example, a touch screen display. In lieu of the foregoing, or in combination therewith, the thermal ablation console 101 may also operably couple to a remote user interface 104. This user interface 104 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

In this example, the thermal ablation console 101 may include an integral control circuit. In lieu of the foregoing, or in combination therewith, the thermal ablation console 101 may operably couple to an external control circuit as denoted by reference numeral 105. When references are made herein to a "control circuit," it will be understood that such a reference can apply to either or both of the foregoing circuits.

Being a "circuit," the control circuit therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit can comprise a fixed-purpose hard-wired hardware platform (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware platform (including but not limited to microcontrollers, microprocessors, and the like). These architectural options for such structures are well known and understood in the art and require no further description here. This control circuit is configured (for example, by using corresponding programming as will be well understood by those skilled in the art) to carry out one or more of the steps, actions, and/or functions described herein.

The control circuit can operably couple to a memory (not shown) if desired. This memory may be integral to the control circuit or can be physically discrete (in whole or in part) from the control circuit as desired. This memory can also be local with respect to the control circuit (where, for example, both share a common circuit board, chassis, power supply, and/or housing) or can be partially or wholly remote with respect to the control circuit (where, for example, the memory is physically located in another facility, metropolitan area, or even country as compared to the control circuit).

Such a memory can serve, for example, to non-transitorily store the computer instructions that, when executed by the control circuit, cause the control circuit to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both non-volatile memory (such as read-only memory (ROM) as well as volatile memory (such as a dynamic random access memory (DRAM).)

If desired the control circuit can also operably couple to a network interface (not shown). So configured the control circuit can communicate with other elements (both within the apparatus 100 and external thereto) via the network interface. Network interfaces, including both wireless and non-wireless platforms, are well understood in the art and require no particular elaboration here.

By one optional approach, and for purposes explained herein, the control circuit operably couple to a thermocouple array 106 and/or to a data acquisition circuit 107. Additional details regarding these components appears further below.

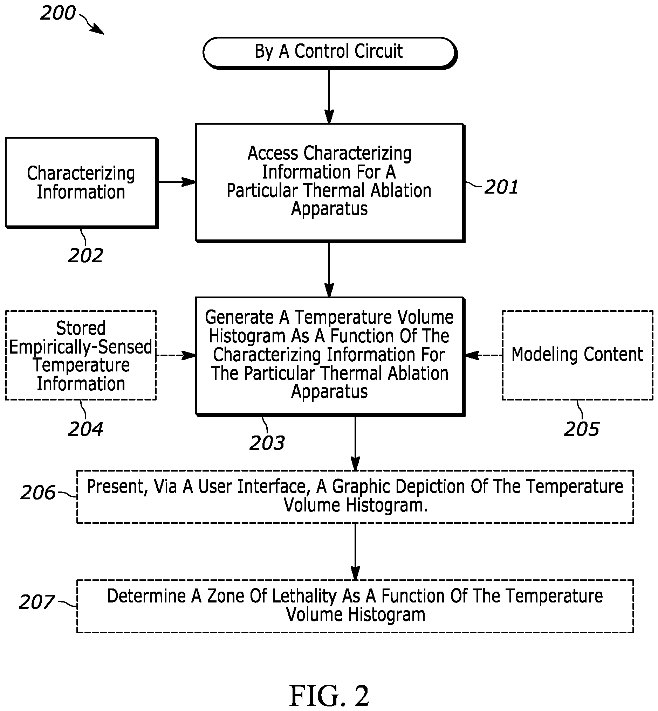

Referring now to FIG. 2, a process 200 that can be carried out, for example, in conjunction with the above-described application setting (and more particularly via the aforementioned control circuit) will be described. Generally speaking, this process 200 serves to improve administration of thermal ablation (including cryotherapy) to a patient's target volume.

At block 201, this process 200 provides for the control circuit to access characterizing information 202 for a particular thermal ablation apparatus (or, optionally, a plurality of different thermal ablation apparatuses). For the sake of a non-limiting illustrative example, the thermal ablation can comprise cryotherapy. A non-exhaustive listing of illustrative examples in these regards for the characterizing information 202 can include information regarding a thermal fluid that effects a desired thermal ablation result, a flow rate for such a thermal fluid, and/or at least one physical dimension of the particular thermal ablation apparatus (such as, but not limited to, a length and/or diameter of a thermal ablation needle/probe).

As suggested above, by one approach the characterizing information 202 for the particular thermal ablation apparatus can comprise characterizing information for a particular thermal ablation needle/probe (such as the probes 102, 103 referenced above). In many application settings, the latter can comprise accessing characterizing information for a particular thermal ablation needle/probe from amongst a plurality of different candidate thermal ablation needles/probes.

At block 203, the control circuit generates a temperature volume histogram as a function of the foregoing characterizing information 202 for the particular thermal ablation apparatus.

A temperature volume histogram represents a three-dimensional temperature distribution in a graphical two-dimensional format. The "volume" refers to the patient volume of interest, such as the patient target volume, a healthy organ located near such a target, an arbitrary structure, and so forth.

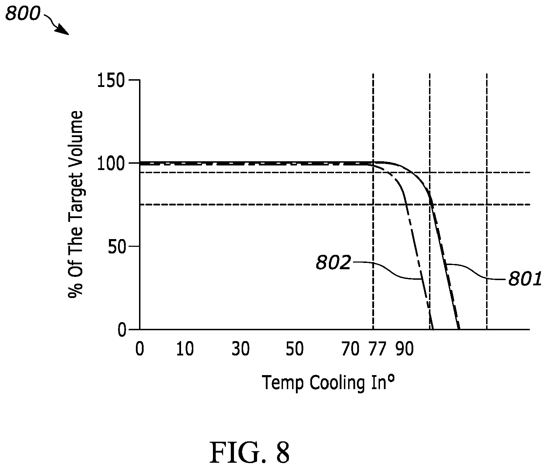

These teachings will accommodate various approaches to presenting a temperature volume histogram. By one approach, the temperature volume histogram can be presented as a differential temperature volume histogram. In this case, a column height for a given temperature bin corresponds to the volume of the structure that corresponds to that temperature. Bin temperatures (comprising or reflecting, for example, relative or absolute temperature variations) typically extend along the horizontal axis while structure volumes (either percent or absolute volumes) extend along the vertical axis. (FIG. 8, discussed further herein, presents an example in these regards.) With high granularity a differntial temperature volume histogram can appear as a smooth line graph.

By another approach, the temperature volume histogram can be presented as a cumulative temperature volume histogram, which can plot bin temperatures along the horizontal axis but has a column height for the first bin that represents the volume of structure(s) that correspond to a temperature greater than or equal to that temperature. The column height of the second bin then represents the volume of structure(s) that correspond to greater than or equal to that temperature, and so forth. With high granularity a cumulative temperature volume histogram can appear as a smooth line graph. (As it happens, FIG. 8 can also serve as an illustrative example of a cumulative temperature volume histogram.)

By one approach, and with reference to optional block 204, generation of the temperature volume histogram can be carried out, at least in part, as a further function of stored empirically-sensed temperature information. That stored empirically-sensed temperature information can correspond, for example, to thermal results of the particular thermal ablation apparatus in a proxy medium. Further information in these regards appears below.

By another approach, in lieu of the foregoing or in combination therewith, the generation of the temperature volume histogram can be carried out, at least in part, as a further function of modeling content 205. Such modeling content 205 can comprise, for example, models of relevant patient thermal behavior or states, patient heat flow, or patient blood flow in or near one or more relevant patient volumes. For example, such modeling content 205 could serve to allow for modifying results that were based on empirically-sensed temperature information as a function of perturbations that derive from a patient's local anatomy (such as blood vessels located near a volume of interest).

These teachings will accommodate taking other factors/information into account as well when generating a temperature volume histogram. For example, generating a temperature volume histogram can also occur as a further function of information regarding a patient's geometry. Examples in these regards include, but are not limited to, specified patient volumes of interest (such as, for example, a tumor volume plus a given margin or a volume of healthy adjacent tissue). By one approach, once such volumes of interest are identified/segmented, one can digitally overlay the expected temperature results for a given needle position. In those regards, generating a temperature volume histogram can occur as a further function of information regarding a particular needle orientation/location/depth with respect to patient geometry and/or a particular plan or pattern of administering and using such treatment accoutrements.

At optional block 206, the control circuit can present (via, for example, a user interface 104 as described above), a graphic depiction of the aforementioned generated temperature volume histogram. That presentation can help, for example, to inform a clinician's planning decisions and/or to facilitate a quality assessment. These teachings will also accommodate providing the user with an opportunity to interact with the temperature volume histogram by, for example, making modifications directly thereto. Those modifications could then be employed by the control circuit to make corresponding modifications to the planned procedure to accord with that desired result. As one example in these regards, a first generated temperature volume histogram could be formed for a first set of presumed operating circumstances (such as a particular choice of needle of fluid, a particular angle of entry and/or depth of the needle). That result could be saved and then the process repeated for a changed set of operating circumstances to yield a second generated temperature volume historgram that the user could then compare to the first generated temperature volume historgram as part of assessing a particular treatment approach to employ.

At optional block 207, the control circuit can determine a zone of lethality as a function of the aforementioned generated temperature volume histogram. That zone of lethality represents areas within one or more patient volumes where the cryotherapy achieves temperatures sufficient to cause cellular death.

The generated temperature volume histogram can accordingly be employed in any of a variety of ways to inform, for example, a thermal ablation treatment for a given patient, which treatment can then be administered to the given patient.

Further details that comport with these teachings will now be presented. It will be understood that the specific details of these examples are intended to serve an illustrative purpose and are not intended to suggest any particular limitations with respect to these teachings.

By one approach, a standard protocol to characterize such things as the aforementioned needles/probes can be defined and/or utilized. Use of a standardized approach can help assure that the characterizing information that is generated for various different needles/probes are not unduly influenced by the particulars of the information gathering design itself (where standardization can be applied, for example, to the choice of the liquid to be frozen, temperature of the sample, ambient temperature control, specifications regarding a thermic recorder, and so forth).

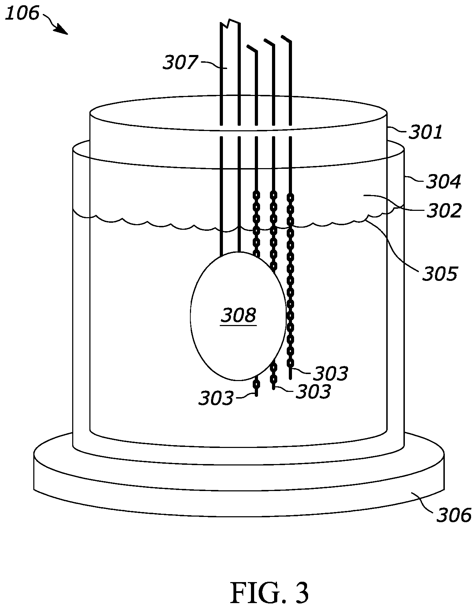

FIG. 3 presents an illustrative representation of a thermocouple array 106 that can accord with these teachings to help develop characterizing information 202. In this example, the thermocouple array 106 comprises a first container 301 having ultrasonic gel 302 (or other suitable matrix of choice) contained therein. An array of thermocouples 303 is disposed through a part or all of that gel 302 (only three lines of thermocouples 303 are shown in FIG. 3 to preserve clarity). The first container 302 is disposed within a second container 304 that has water 305 disposed therein to form a warm water bath for the gel 302. That second container 304 rests atop a hot plate 306. So configured, the gel 302 can be heated to a temperature that is within the range of normal human body temperature, such as 37 degrees Celsius.

A cryotherapy needle/probe 307 can be placed into the gel and held in place while characterizing information is collected. As one example, the cryotherapy needle/probe 307 can be used at a particular standardized power level (such as full power) for a particular standardized period of time (such as five minutes). An expected ice ball 308 will form in the gel 302. The temperatures achieved within the gel 302 by the foregoing are sensed by the various thermocouples and collected, for example, by the aforementioned data acquisition circuit 107.

The generated data may be delivered as an absolute temperature per voxel. The size of the voxel can be determined by the initial temperature recording system (for example, a cube having a cross section of 2.7mm). When this absolute temperature is provided as a negative value, that value can be converted into a positive value. For example, one can use the absolute cooling temperature, which is equal to the initial temperature (37 degrees Celsius in the foregoing example) minus the recorded temperature.

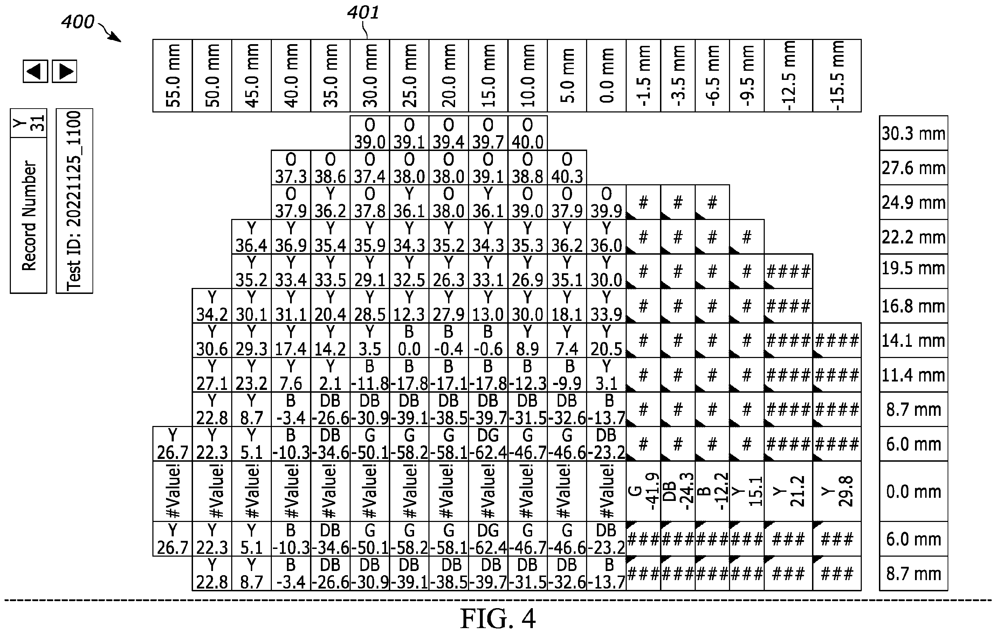

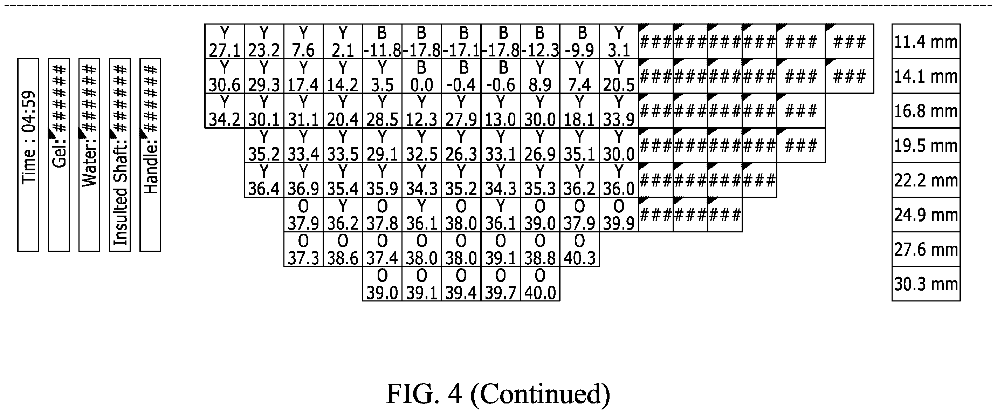

FIG. 4 presents an illustrative example of a resultant isotherm map 400 at a desired resolution in accord with the foregoing. Color can be used to highlight voxels having a temperature within a corresponding range. In this figure, the letters "DG" symbolize a dark green color, the letter "G" symbolizes a green color, the letters "DB" symbolize a dark blue color, the letter "B" symbolizes a blue color, the letter "Y" symbolizes a yellow color, and the letter "O" symbolizes an orange color.

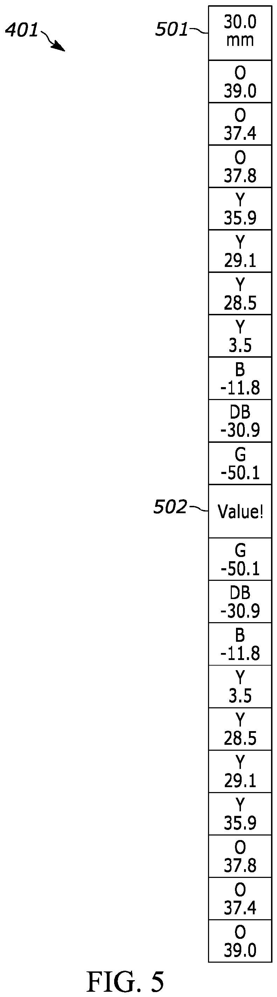

These teachings will also accommodate depicting, in some or all of these voxel boxes, the corresponding temperature. As an illustrative example, FIG. 5 depicts an enlarged view of the row denoted by the reference numeral 401 in the foregoing isotherm map 400. The box at the far right and denoted by reference numeral 501 identifies a particular distance that corresponds to the needle/probe itself. A box in the center that is denoted by reference numeral 502 refers to the cross section of the needle/probe. The remaining boxes not only have a corresponding color as described above, but also present a corresponding temperature as described above.

In many cases, the foregoing voxels can be reduced in size in order to achieve a clinically relevant voxel size (for example, a volume at or about 1mm³). By one approach, a mathematical transformation can serve to transform a single voxel of 2.7mm³ into 27 voxels of 0.9mm³.

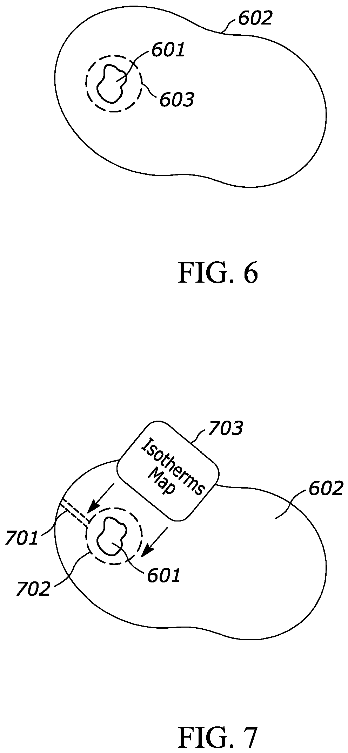

By way of a more specific illustrative example, FIG. 6 presents a tumor 601 in a patient 602. The boundary of the tumor 601 defines the gross tumor volume. Clinical guiding images such as this can be obtained using computed tomography, magnetic resonance imaging, or any other suitable imaging methodology of choice. The dashed line 603 encircling the gross tumor volume is the clinical tumor volume 603. Defining a clinical tumor volume comprises a well-understood area of prior art endeavor and accordingly needs no further elaboration here.

FIG. 7 depicts a particular needle/probe injection path 701 and the expected extent of a corresponding ice ball 702. The aforementioned three-dimensional isotherms map 703 of absolute cooling temperatures can be digitalized and registered over this clinical guiding image.

In this regard, a treatment planning system can provide useful support. Using such a system, the target volume and other regions of interest can be captured, displayed, and segmented to identify their contours. These volumes can be displayed in mm³ or any other relevant metric. By one approach, the treatment planning system can display, in a live setting, the appearance/location of the selected needle/probe(s). In addition, the expected ice ball, according to the measures calculated above, can also be presented. Such a presentation can identify such metrics as the above-described absolute cooling temperatures, which can be, for example, the minimal absolute cooling temperature required to generate an ice ball. These teachings will also accommodate allowing the user to select other values for the absolute cooling temperature, and to generate their display in a three-dimensional rendition (based on generated data described above).

FIG. 8 presents an illustrative example of a temperature volume histogram 800 that could correspond to the foregoing. The solid line 801 corresponds to the clinical tumor volume while the dashed line 802 corresponds to the gross tumor volume. Note that the Y-axis can be displayed as the absolute volume, as a percentage of the volume, or any other metric relevant for volume evaluation. The X-axis, in turn, can be displayed as an absolute temperature variation such as, in this example using cold temperatures to effect thermal ablation, the absolute cooling temperature or any other temperature value that can serve to translate the differential between the resting temperature of the target (typically around 37 degrees Celsius for a human being) and the post or intra-treatment temperature.

By one approach, the aforementioned data (including the corresponding temperature volume histograms) can be exportable. If desired, both the final temperatures achieved and the time-longitudinal data can be exportable. The availability of these data can enable, for example, fine tuning of the approaches used to estimate the cooling process for each needle/probe as a function of the patient's overall anatomy.

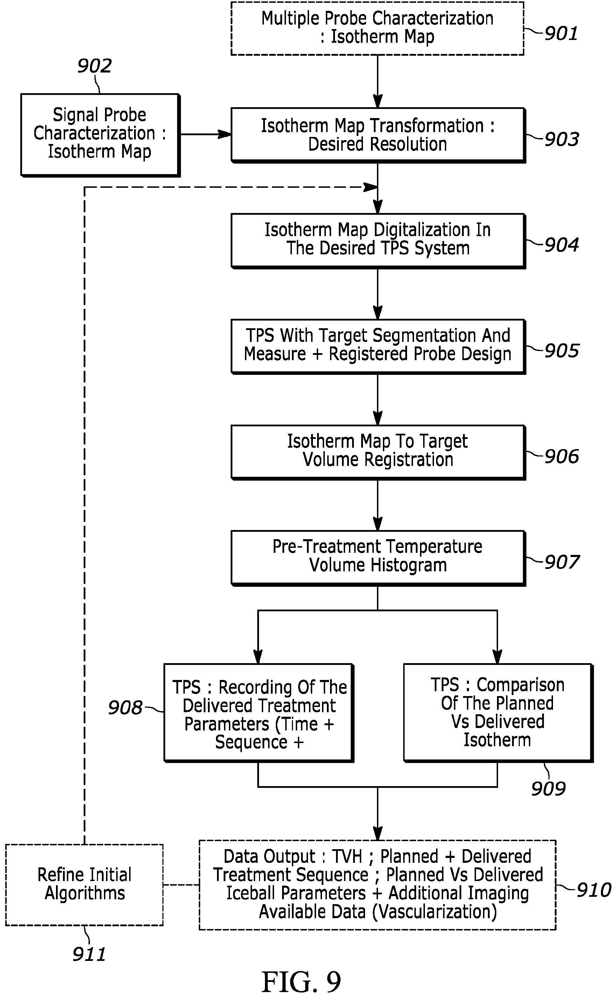

Referring now to FIG. 9, an example in accord with these teachings that provides for data recovery and that can support, if desired, the use of artificial intelligence-based correction of an expected isotherm pattern/ice ball will be described.

At block 901, a plurality of thermal ablation needles/probes are characterized with corresponding isotherm maps (for example, as described above).

At block 903, this process transforms an isotherm map 902 for a particular needle/probe to a desired resolution (again, and for example, as described above).

At block 904, this process digitizes the transformed isoform map for use with a particular treatment planning system.

At block 905, this process utilizes the treatment planning system to automatically segment, measure, and register the selected needle/probe 902 and at block 906 the isotherm map is registered with the target volume.

At block 907, this process generates a pre-treatment temperature volume histogram. At block 908, the treatment planning system records the delivered treatment parameters (including such parameters as time and sequence of steps). At block 909 the treatment planning system compares the planned isotherm information with the delivered isotherm information.

At block 910, the process provides for outputting various data items. Examples include, but are not limited to, one or more relevant thermal volume histograms, the planned and the delivered sequence, the planned and the delivered ice ball(s) parameters, and other information desired (such as imaging information and other information regarding such things as vascularization).

Based on at least the latter, at block 911 this process refines one or more initial algorithms that were applied in earlier steps. Such feedback can be limited as desired, with the feedback only being applied, for example, on a per-patient basis, a per-practitioner basis, a per-clinic basis, a per-institution basis, or so forth.

Further aspects of the invention are provided by the subject matter of the following clauses:
Clause 1. A method to facilitate administering thermal ablation to a patient's target volume, the method comprising: by a control circuit: accessing characterizing information for a particular thermal ablation apparatus; generating a temperature volume histogram as a function of the characterizing information for the particular thermal ablation apparatus.
Clause 2. An apparatus to facilitate administering thermal ablation to a patient's target volume, the apparatus comprising: a memory having stored therein characterizing information for a particular thermal ablation apparatus; a control circuit operably coupled to the memory and configured to: access the characterizing information for the particular thermal ablation apparatus; and generate a temperature volume histogram as a function of the characterizing information for the particular thermal ablation apparatus.
Clause 3. Any combination of the clauses presented herein wherein accessing the characterizing information for the particular thermal ablation apparatus comprises accessing characterizing information for a particular thermal ablation needle.
Clause 4. Any combination of the clauses presented herein wherein accessing the characterizing information for the particular thermal ablation needle comprises accessing characterizing information for the particular thermal ablation needle from amongst a plurality of different candidate thermal ablation needles.
Clause 5. Any combination of the clauses presented herein wherein the thermal ablation comprises cryotherapy.
Clause 6. Any combination of the clauses presented herein further comprising: by the control circuit: determining a zone of lethality as a function of the temperature volume histogram.
Clause 7. Any combination of the clauses presented herein further comprising: by the control circuit: presenting, via a user interface, a graphic depiction of the temperature volume histogram.
Clause 8. Any combination of the clauses presented herein wherein generating the temperature volume histogram further comprises generating the temperature volume histogram as a further function of stored empirically-sensed temperature information.
Clause 9. Any combination of the clauses presented herein wherein the stored empirically-sensed temperature information corresponds to thermal results of the particular thermal ablation apparatus in a proxy medium.
Clause 10. Any combination of the clauses presented herein wherein the characterizing information for the particular thermal ablation apparatus includes at least one of: a thermal fluid that effects a desired thermal ablation result; a flow rate of the thermal fluid; and at least one physical dimension of the particular thermal ablation apparatus.
Clause 11. Any combination of the clauses presented herein wherein generating the temperature volume histogram further comprises generating the temperature volume histogram as a further function of modeling content.
Clause 12. Any combination of the clauses presented herein wherein the modeling content represents at least one of patient thermal states, patient blood flow, and patient anatomy.
Clause 13. Any combination of the clauses presented herein wherein generating the temperature volume histogram further comprises generating the temperature volume histogram as a further function of information regarding a patient's geometry.
Clause 14. Any combination of the clauses presented herein wherein the information regarding the patient's geometry comprises at least one of a specified patient volume of interest, a particular thermal ablation needle orientation/location/depth with respect to patient geometry, and/or a particular plan or pattern of administering and using treatment accoutrements.

Those skilled in the art will recognize that a wide variety of modifications, alterations, and combinations can be made with respect to the above described embodiments without departing from the scope of the invention. As one example, these teachings will accommodate evaluating the aforementioned temperature mapping and/or presentation/use of the temperature volume histogram during planification as the needle/probe insertion axis is changed by the practitioner and/or during formation (or thawing) of an ice ball. To support the foregoing, the generated data can be timely longitudinal over several minutes. Such modifications, alterations, and combinations are therefore to be viewed as being within the ambit of the inventive concept.

## Claims

1. A method to facilitate administering thermal ablation to a patient's target volume, the method comprising:
by a control circuit (105):
accessing characterizing information for a particular thermal ablation apparatus (201);
generating a temperature volume histogram as a function of the characterizing information for the particular thermal ablation apparatus (203), optionally accessing the characterizing information (202) for the particular thermal ablation apparatus comprises accessing characterizing information for a particular thermal ablation needle as a further option accessing the characterizing information for the particular thermal ablation needle comprises accessing characterizing information for the particular thermal ablation needle (102) from amongst a plurality of different candidate thermal ablation needles (103) wherein optionally the thermal ablation comprises cryotherapy.

2. The method of claim 1 further comprising:
by the control circuit:
determining a zone of lethality as a function of the temperature volume histogram (207).

3. The method of claim 1 or 2 further comprising:
by the control circuit:
presenting, via a user interface, a graphic depiction of the temperature volume histogram (206).

4. The method of any one of the preceding claims, wherein generating the temperature volume histogram further comprises generating the temperature volume histogram as a further function of stored empirically-sensed temperature information (204), wherein optionally the stored empirically-sensed temperature information corresponds to thermal results of the particular thermal ablation apparatus in a proxy medium.

5. The method of any one of the preceding claims, wherein the characterizing information for the particular thermal ablation apparatus includes at least one of:
a thermal fluid that effects a desired thermal ablation result;
a flow rate of the thermal fluid; and
at least one physical dimension of the particular thermal ablation apparatus.

6. The method of any one of the preceding claims, wherein generating the temperature volume histogram further comprises generating the temperature volume histogram as a further function of modeling content (205), wherein optionally the modeling content represents at least one of patient thermal states, patient blood flow, and patient anatomy.

7. The method of any one of the preceding claims, wherein generating the temperature volume histogram further comprises generating the temperature volume histogram as a further function of information regarding a patient's geometry, wherein optionally the information regarding the patient's geometry comprises at least one of a specified patient volume of interest, a particular thermal ablation needle orientation/location/depth with respect to patient geometry, and/or a particular plan or pattern of administering and using treatment accoutrements.

8. An apparatus to facilitate administering thermal ablation to a patient's target volume, the apparatus comprising:
a memory having stored therein characterizing information for a particular thermal ablation apparatus (102, 103);
a control circuit (105) operably coupled to the memory and configured to:
access the characterizing information for the particular thermal ablation apparatus; and
generate a temperature volume histogram as a function of the characterizing information for the particular thermal ablation apparatus.

9. The apparatus of claim 8 wherein the control circuit is configured to access the characterizing information for the particular thermal ablation apparatus by accessing characterizing information for a particular thermal ablation needle, wherein optionally accessing the characterizing information for the particular thermal ablation needle comprises accessing characterizing information for the particular thermal ablation needle (102) from amongst a plurality of different candidate thermal ablation needles (103), optionally the thermal ablation comprises cryotherapy.

10. The apparatus of claim 8 or 9 wherein the control circuit is further configured to determine a zone of lethality as a function of the temperature volume histogram.

11. The apparatus of any one of claims 8-10, further comprising:
a user interface (104) that is operably coupled to the control circuit (105); and
wherein the control circuit is further configured to present, via the user interface, a graphic depiction of the temperature volume histogram.

12. The apparatus of any one of claims 8-11 wherein the control circuit is further configured to generate the temperature volume histogram as a further function of stored empirically-sensed temperature information wherein optionally, the stored empirically-sensed temperature information corresponds to thermal results of the particular thermal ablation apparatus in a proxy medium.

13. The apparatus of any one of claims 8-12 wherein the characterizing information for the particular thermal ablation apparatus includes at least one of:
a thermal fluid that effects a desired thermal ablation result;
a flow rate of the thermal fluid; and
at least one physical dimension of the particular thermal ablation apparatus.

14. The apparatus of any one of claims 8-13 wherein the control circuit is further configured to generate the temperature volume histogram by generating the temperature volume histogram as a further function of modeling content (205) , wherein optionally the modeling content represents at least one of patient thermal states, patient blood flow, and patient anatomy.

15. The apparatus of any one of claims 8-14 wherein the control circuit is further configured to generate the temperature volume histogram by generating the temperature volume histogram as a further function of information regarding a patient's geometry wherein optionally the information regarding the patient's geometry comprises at least one of a specified patient volume of interest, a particular thermal ablation needle orientation/location/depth with respect to patient geometry, and/or a particular plan or pattern of administering and using treatment accoutrements.
